# EUROPEAN PATENT SPECIFICATION

(11) **EP 1 761 266 B1**
(45) Date of publication and mention of the grant of the patent: **08.05.2013**
(21) Application number: 05754754.9
(22) Date of filing: 25.05.2005
(51) Int. Cl.: A61K 31/498, A61K 31/4164, A61P 17/00

(54) **COMPOUNDS, FORMULATIONS, AND METHODS FOR TREATING OR PREVENTING INFLAMMATORY SKIN DISORDERS**
VERBINDUNGEN, FORMULIERUNGEN UND VERFAHREN ZUR BEHANDLUNG ODER PRÄVENTION VON ENTZÜNDLICHEN HAUTERKRANKUNGEN
COMPOSES, PREPARATIONS ET METHODES PERMETTANT DE TRAITER OU DE PREVENIR DES TROUBLES INFLAMMATOIRES CUTANES

(30) Priority: 25.05.2004 US 853585; 25.05.2004 US 574142 P
(43) Date of publication of application: 14.03.2007
(60) Divisional application: 12165557.5
(73) Proprietor: Galderma Pharma S.A., 1000 Lausanne 30 Grey (CH)
(72) Inventor: DEJOVIN, Jack A., New Brunswick, New Jersey 08901-1515 (US); ROSSI, Thomas M., Stockton, New Jersey 08559-2009 (US)
(74) Representative: Jansen, Cornelis Marinus
(86) International application number: PCT/US2005/018288
(87) International publication number: WO 2005/115395

(56) References cited:
- WO-A-2004/105703
- WO-A-2005/002580
- WO-A-2005/010025
- US-A- 4 256 763
- US-A- 4 285 967
- US-A- 5 720 962
- US-A- 5 916 574
- US-A- 6 117 877
- GUARRERA M ET AL: "FLUSHING IN ROSACEA: A POSSIBLE MECHANISM" ARCHIVES OF DERMATOLOGICAL RESEARCH, SPRINGER, INTERNATIONAL, BERLIN, DE, vol. 272, no. 3/04, 1 January 1982 (1982-01-01), pages 311-316, XP008012346 ISSN: 0340-3696
- REBORA A: "THE MANAGEMENT OF ROSACEA" AMERICAN JOURNAL OF CLINICAL DERMATOLOGY, ADIS, US, vol. 3, 1 January 2002 (2002-01-01), pages 489-496, XP009034164 ISSN: 1175-0561
- BURKE J ET AL: "Preclinical evaluation of brimonidine" SURVEY OF OPHTHALMOLOGY, SURVEY OF OPHTHALMOLOGY INC, XX, vol. 41, 1 November 1996 (1996-11-01), pages S9-S18, XP023437195 ISSN: 0039-6257 [retrieved on 1996-11-01]
- NIELSEN H ET AL: "POSTJUNCTIONAL ALPHA-2-ADRENOCEPTORS MEDIATE VASOCONSTRICTION IN HUMAN SUBCUTANEOUS RESISTANCE VESSELS" BRITISH JOURNAL OF PHARMACOLOGY, vol. 97, no. 3, 1989, pages 829-834, XP009114426 ISSN: 0007-1188
- LINDGREN B R ET AL: "EFFECTS OF SOME ANTIHYPERTENSIVE DRUGS ON CUTANEOUS BLOOD FLOW AND INFLAMMATORY SKIN RESPONSES FOLLOWING ALLERGEN CHALLENGE IN GUINEA-PIGS" PHARMACOLOGY AND TOXICOLOGY, vol. 60, no. 5, 1987, pages 364-367, XP002546987 ISSN: 0901-9928

## Description

### Field Of The Invention

The present invention is directed to compositions for use in methods for the topical treatment or prevention of inflammatory skin disorders as defined in appended claim 1. The compounds and methods taught by the present invention are particularly useful for treating or preventing the inflammatory skin disorders and the symptoms associated therewith.

### Background Of The Invention

Many people are affected by inflammatory skin disorders that result in unsightly and painful rashes, acne, persistent red veins, and acne-like skin eruptions, such as macules, nodules, and pustules that may ooze or crust. Inflammatory skin disorders often result in intense psychosocial distress. Rosacea is a common inflammatory skin disorder affecting over 10 million people in the United States. Rosacea generally involves the cheeks, nose, chin, and forehead and the typical age of onset is 30 to 60 years. See e.g., Zuber T.J., Rosacea: Beyond First Blush 32 HOSP. PRACT. 188-189 (1997); THE MERCK MANUAL 813-814 (Keryn A.G. Lane et al. eds. 17th ed. 2001). Many people with early-stage rosacea incorrectly assume that they suffer from adult acne, sun or windburn, or the normal effects of aging.

Rosacea develops gradually starting as frequent blushing and frequent irritation of the facial skin.

More advanced rosacea is characterized by a vascular stage where patients display increasingly severe erythema (abnormal redness of the skin) and telangiectasia (visible red lines due to abnormal dilatation of capillary vessels and arterioles). Pimple-like eruptions, which may be solid (called papules or nodules) or puss filled (known as pustules) may develop. Such eruptions often look like acne, but whiteheads or blackheads (common symptoms of acne) are not normally present. Later-stage rosacea is characterized by rhinophyma (enlargement of the nose). If left untreated, rosacea can progress to irreversible disfigurement. Rosacea symptoms are often aggravated by sun exposure, changes or extremes in temperature, wind, and consumption of certain foods, such as spicy foods, caffeine, and alcohol.

The exact pathogenesis of rosacea is unknown, but the pathologic process is well described. For example, erythema associated with rosacea is caused by dilation of the superficial vasculature of the face. Zuber T.J., Rosacea: Beyond First Blush 32 HOSP. PRACT. 188-189 (1997).

There is no known cure for many inflammatory skin disorders like rosacea. Current treatments, which are directed to control of redness, inflammation, and skin eruptions, are of limited effectiveness in many patients and, generally, can be used only for a limited duration. Standard treatments include avoidance of triggers such as sun exposure, wind exposure, alcohol consumption, spicy foods, and irritating facial cleansers, lotions, and cosmetics. Antibiotics are the traditional first line of therapy. Long-term treatment (5 to 8 weeks or more) with oral antibiotics such as tetracycline, minocycline, doxycycline or clarithromycin may control skin eruptions. Alternative oral treatments include vitamin A medications, such as isoretinoin and antifungal medications. Unfortunately, such oral medications often cause side effects and many people have limited tolerance. Topical treatments, such at topically applied antibiotics and antifungals (such as metronidazole) or steroids, are available but also have limited effectiveness and cannot treat all symptoms. For example, isoretinoin has serious teratogenic side-effects and female patients of child bearing age must use effective birth control or avoid the therapy. Topical treatments include topically applied metronidazole, topically applied steroids, topically applied azelaic acid, topically applied rentinoic acid or retinaldehyde, and topical vitamin C preparations are available but have limited effectiveness and cannot treat all symptoms. Surgery, such as the laser elimination of blood vessels, is typically a last resort, but may be prescribed if other treatments are ineffective. In patients with nose hyperplasia, surgical reduction may improve the patient's cosmetic appearance, but does not treat the disease itself. Mixed light pulse (photoderm) therapy has proved somewhat effective for symptoms associated with certain inflammatory skin orders like rosacea in some patients. Thus, there remains a need for topical formulations for treatment of inflammatory skin disorders like rosacea and its symptoms.

Agonists of the α₂ adrenoceptors have been used therapeutically for a number of conditions including hypertension, congestive heart failure, angina pectoris, spasticity, glaucoma, diarrhea, and for the suppression of opiate withdrawal symptoms (J.P. Heible and R.R. Ruffolo Therapeutic Applications of Agents Interacting with α-Adrenoceptors, p. 180-206 in Progress in Basic and Clinical Pharmacology Vol. 8, P. Lomax and E.S. Vesell Ed., Karger, 1991).

Adrenoreceptor agonists such as clonidine have been primarily used orally, though a patch formulation is known. The goal of existing formulations is to deliver a systemic internal dose of the compound to the patient. The α₂ agonists are known to mediate vasoconstriction both in the core and periphery of a patient. In particular α₂ adrenoceptor agonists are known to cause vasoconstriction of peripheral arterioles, in response to stimulation due to cold or stress.

A number of patents describe the use of brimonidine for treating ophthalmic conditions and eye diseases. In Canadian patent No. CA2326690, there is described the use of topical ophthalmic preparations for use only in the eyes, to treat eye diseases. The Canadian patent discusses the problems with ophthalmic preparations taken topically (in the eye), orally or parenterally, and their systemic effects, including some serious, that limit their use. These systemic effects include, cardiopulmonary effects of β-blockers like timolol; dryness of mouth, flush, fever, tachy cardia, urinary retention, convulsion and irritability with atropine; hypertension with phenylephine; increased salivation, nausea, vomiting, diarrhea, stomach cramps, bronchial secretions, bronchial constriction, asthma, bradycardia, paresthesia with miotics; hypotension with clonidine; and dry mouth, fatigue and drowsiness with apraclonidine and brimonidine.

There has been no composition containing α₂ adrenoceptor agonists that can deliver a dose of the agonist to the patient, ameliorating the symptoms of rosacea or other inflammatory skin disorders, without causing systemic side effects. There has also been no topical skin composition containing α₂ adrenoceptor agonists that can deliver a dose of the agonist to the skin of the patient, ameliorating the symptoms of rosacea and/or other inflammatory skin disorders, without causing systemic side effects.

WO 2005/002580 discloses a method of preventing or reducing the severity of a stress- associated inflammatory dermatological condition in a subject by systemically administering brimonidine or a pharmaceutically acceptable salt, ester, amide, stereoisomer or racemic mixture thereof. The European patent application (publication No. EP 1638569) derived from the foregoing international patent application is comprised in the state of the art within the meaning of Article 54(3) EPC.

US-A- 4 256 763 discloses a combination comprising phenolphthalein and phenylephrine for the treatment of acne, whereby the combination is administered orally.

US-A-5 916 574 discloses a natural poison skin treatment composition which includes (i) a natural poison skin treatment medication selected from benzoyl peroxide and salicyclic acid; (ii) a vasoconstrictor selected from, amongst others, catecholamines, norepinephrine, epinephrine, isoproterenol, dopamine, ephedrine, phenylisopropoylamines, phenylephrine, amphetamine, metraminol, methoxamine, lysergic acid, and lysergic acid diethyamine; and (iii) an inert carrier. The vasoconstrictor is stated to be used to remove the redness associated with the natural poison skin condition, such as poison oak, poison ivy and poison sumac.

US-A-4 285 967 discloses cosmetic compositions comprising phenylephrine hydrochloride for reducing the redness of facial blemishes.

Lindgren B. R. et al.: "Effects of some antihypertensive drugs on cutaneous blood flow and inflammatory skin responses following allergen challenge in guinea-pigs", Pharmacology and Toxicology, Vol. 60, No. 5, 1987, pp. 364-367 describes the result of a study undertaken to investigate the effects of the antihypertensive drugs, clondine, prazosin and MK 422, on cutaneous blood flow and allergen-induced inflammatory skin responses. The tests involved administering the drugs by intraperitonial injections twice a day.

### Summary Of The Invention

The present invention provides compositions and topical skin formulations for the treatment of inflammatory skin disorders and their symptoms in accordance with appended claims 1 and 2. compounds underlying the compositions of the invention are particularly effective for treatment of the inflammatory skin diseases dermatitis, such as contact dermatitis, atopic dermatitis, seborrheic dermatitis, nummular dermatitis, generalized exfoliative dermatitis, statis dermatitis, lichen simplex chronicus; disorders of hair follicles and sebaceous glands, such as acne, rhinophyma, perioral dermatitis, and pseudofolliculitis barbae; and inflammatory reactions, such as drug eruptions, erythema multiforme, erythema nodosum, and granuloma annulare. Compounds underlying the compositions of the invention are α₂ adrenoceptor agonists that act on the peripheral vasculature to cause vasoconstriction and thereby ameliorate the symptoms of inflammatory skin disorders. The compounds are delivered in a topical skin composition that insures that the compounds are effective in the skin of a patient but do not penetrate the skin in sufficient amounts to induce serious systemic side effects.

Compounds underlying the compositions of the invention are selected from brimonidine, oxymetazoline and their respective pharmaceutically acceptable salts.

To treat or prevent inflammatory skin disorders according to the invention, the compositions of the invention are topically applied. Preferably the compounds are delivered in a topical formulation. Formulations for topical delivery of compounds of the invention are well-known in the art, such as aqueous or non-aqueous solutions or suspensions, creams, lotions, gels, or ointments.

These and other features, aspects, and advantages of the invention will become better understood with reference to the following detailed description, examples, and appended claims.

### Detailed Description

### 1.1 COMPOUND UNDERLYING THE COMPOSITIONS OF THE INVENTION

The α₂ adrenergic receptor agonist and/or a pharmaceutically acceptable salt thereof underlying the compositions of the present

Compounds underlying the compositions of the invention are listed in Table 1 below.

**Table 1: Compounds Of The Invention**

| Compound of the Invention | Name |
|---|---|
| 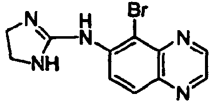 | (5-Bromo-quinoxalin-6-yl)-(4,5-dihydro-1H-imidazol-2-yl)-amine(Brimonidine) |
| 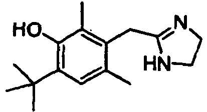 | Oxymetazoline |

The most preferred compound is (5-Bromo-quinoxalin-6-yl)-(4,5-dihydro-1H-imidazol-2-yl)-amine (commonly referred to as brimonidine) and pharmaceutically acceptable salts thereof, particularly the tartrate salt.

The compounds underlying the compositions of the invention are well known in the art to be α₂ adrenergic receptor agonists. As such the compounds have powerful vasoconstricting effects when introduced into the body of mammals, particularly humans.

### 1.2 SYNTHESIS OF COMPOUNDS UNDERLYING THE COMPOSITIONS OF THE INVENTION

The compounds described above can be prepared in accordance with well-known synthetic procedures, for example, using the general synthetic procedures outlined in U.S. Patent Nos. 3,890,319 (issued June 17, 1975) and 4,029,792 (issued June 14, 1977). Scheme 1 below illustrates one method to synthesize compounds of Formula **I,** brimonidine being such a compound: one of R₁, R₂ and R₃ provides for the bromo substituent, the remaining groups, as well as R₄ to R_{7,} are all hydrogen.

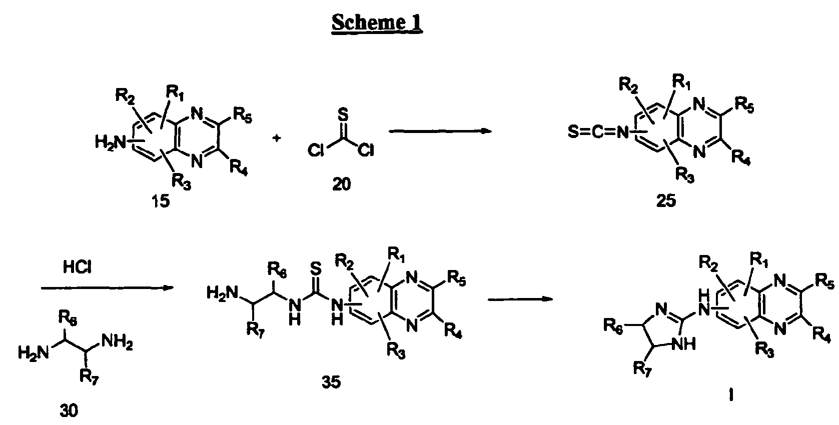

The appropriate quinoxaline **15** is reacted with thiophosgene 20 to form the corresponding isothiocyanate 25. The reaction with thiophosgene can be carried out in aqueous solution or in dilute aqueous hydrochloric acid at room temperature in a period of about 2 hours. Alternatively, the thiophosgene 20 dissolved in a water-immiscible solvent, such as chloroform, can be added to a basic aqueous solution (sodium carbonate) of the quinoxaline 15 and stirred for about two hours. In the first alternative, the isothiocyanate 25 precipitates from the reaction mixture. Precipitation can be completed by neutralization with excess aqueous base. Precipitated isothiocyanate 25 is recovered by filtration and dissolved in a suitable solvent, e.g., chloroform, to form a solution. The solution is dried (e.g., MgSO₄), filtered, and concentrated to yield the isothiocyanate **25**.

Isothiocyanate 25 is treated with an excess of the appropriately substituted ethylene diamine 30 to form the corresponding 3-quinoxalin-6-yl-thiourea 35. Isothiocyanate 25 is reacted with an excess (e.g., 5 moles to 1 mole) of ethylene diamine 30 in a suitable solvent, e.g., diethyl ether, benzene, chloroform or dioxane.
The reaction is carried out at room temperature for about 2 hours. The 3-Quinoxalin-6-yl-thiourea 35 precipitates and is recovered by filtration and washing the filter cake with solvent.

Cyclization of the 3-quinoxalin-6-yl-thiourea **35** to afford compounds for use in the compositions of the invention is effected by heating a suspension of the thiourea **35** with mercuric or cupric oxide in a suitable organic solvent, e.g., ethanol. The mercuric or cupric oxide can be replaced by an organic soluble mercuric or cupric salt, e.g., mercuric or cupric acetate. The reaction mixture is filtered, to remove the mercuric or cupric sulfide by-product, and the filtrate is concentrated to give compound **10** in crude form. Compound **10** is recrystallized as the free base or converted to an acid-addition salt by conventional reaction with a suitable acid. In certain cases, cyclization can be effected by simply refluxing the thiourea **35** in a suitable organic solvent, e.g., methanol, in the absence of mercuric or cupric oxide.

The quinoxaline **15** is synthesized by well-known synthetic procedures, for example, the procedures disclosed in J.A. JOULE ET AL., HETEROCYCLIC CHEMISTRY 189-224. (3rd ed. 1995).

### 1.3 TOPICAL FORMULATIONS OF THE INVENTION

The compounds described above are delivered to the affected area of the skin in a pharmaceutically acceptable topical carrier. As used herein, a pharmaceutically acceptable topical carrier is any pharmaceutically acceptable formulation that can be applied to the skin surface for topical, dermal, intradermal, or transdermal delivery of a pharmaceutical or medicament. The combination of a pharmaceutically acceptable topical carrier and a compound described above is termed a topical formulation of the invention. Topical formulations of the invention are prepared by mixing a compound described above with a topical carrier according to well-known methods in the art, for example, methods provided by standard reference texts such as, REMINGTON: THE SCIENCE AND PRACTICE OF PHARMACY 1577-1591, 1672-1673, 866-885(Alfonso R. Gennaro ed. 19th ed. 1995); Ghosh, T. K; et al. TRANSDERMAL AND TOPICAL DRUG DELIVERY SYSTEMS (1997).

The topical carriers useful for topical delivery of compounds described above can be any carrier known in the art for topically administering pharmaceuticals, for example, but not limited to, pharmaceutically acceptable solvents, such as a polyalcohol or water, emulsions (either oil-in-water or water-in-oil emulsions), such as creams or lotions; micro emulsions; gels; ointments; liposomes; powders; and aqueous solutions or suspensions, such as standard ophthalmic preparations.

### 1.3.1 Emulsions, Gels, and Ointments As Topical Carriers

In a preferred embodiment, the topical carrier used to deliver a compound described above is an emulsion, gel, or ointment. Emulsions such as creams and lotions are suitable topical formulations for use in accordance with the invention. An emulsion is a dispersed system comprising at least two immiscible phases, one phase dispersed in the other as droplets ranging in diameter from 0.1 µm to 100 µm. An emulsifying agent is typically included to improve stability. When water is the dispersed phase and an oil is the dispersion medium, the emulsion is termed a water-in-oil emulsion. When an oil is dispersed as droplets throughout the aqueous phase as droplets, the emulsion is termed an oil-in-water emulsion. Emulsions, such as creams and lotions that can be used as topical carriers and their preparation are disclosed in REMINGTON: THE SCIENCE AND PRACTICE OF PHARMACY 282-291 (Alfonso R. Gennaro ed. 19th ed. 1995).

In another embodiment, the topical carrier used to deliver a compound described above is a gel, for example, a two-phase gel or a single-phase gel. Gels are semisolid systems consisting of suspensions of small inorganic particles or large organic molecules interpenetrated by a liquid. When the gel mass comprises a network of small discrete inorganic particles, it is classified as a two-phase gel. Single-phase gels consist of organic macromolecules distributed uniformly throughout a liquid such that no apparent boundaries exist between the dispersed macromolecules and the liquid. Suitable gels for use in the invention are disclosed in REMINGTON: THE SCIENCE AND PRACTICE OF PHARMACY 1517-1518 (Alfonso R. Gennaro ed. 19th ed. 1995). Other suitable gels for use in the invention are disclosed in U.S. Patent Nos. 6,387,383 (issued May 14, 2002); 6,517,847 (issued Feb. 11, 2003); and 6,468,989 (issued Oct. 22, 2002).

Polymer thickeners (gelling agents) that may be used include those known to one skilled in the art, such as hydrophilic and hydroalcoholic gelling agents frequently used in the cosmetic and pharmaceutical industries. Preferably, the hydrophilic or hydroalcoholic gelling agent comprises "CARBOPOL®" (B.F. Goodrich, Cleveland, Ohio), "HYPAN®" (Kingston Technologies, Dayton, N.J.), "NATROSOL®" (Aqualon, Wilmington, Del.), "KLUCEL®" (Aqualon, Wilmington, Del.), or "STABILEZE®" (ISP Technologies, Wayne, N.J.). Preferably the gelling agent comprises between about 0.2% to about 4% by weight of the composition. More particularly, the preferred compositional weight percent range for "CARBOPOL®" is between about 0.5% to about 2%, while the preferred weight percent range for "NATROLSOL®" and "KLUCEL®" is between about 0.5% to about 4%. The preferred compositional weight percent range for both "HYPAN®" and "STABILEZE®" is between 0.5% to about 4%.

"CARBOPOL®" is one of numerous cross-linked acrylic acid polymers that are given the general adopted name carbomer. These polymers dissolve in water and form a clear or slightly hazy gel upon neutralization with a caustic material such as sodium hydroxide, potassium hydroxide, triethanolamine, or other amine bases. "KLUCEL®" is a cellulose polymer that is dispersed in water and forms a uniform gel upon complete hydration. Other preferred gelling polymers include hydroxyethylcellulose, cellulose gum, MVE/MA decadiene crosspolymer, PVM/MA copolymer, or a combination thereof.

In another preferred embodiment, the topical carrier used to deliver a compound described above is an ointment. Ointments are oleaginous semisolids that contain little if any water. Preferably, the ointment is hydrocarbon based, such as a wax, petrolatum, or gelled mineral oil. Suitable ointments for use in the invention are well known in the art and are disclosed in REMINGTON: THE SCIENCE AND PRACTICE OF PHARMACY 1585-1591 (Alfonso R. Gennaro ed. 19th ed. 1995).

### 1.3.2 Aqueous Topical Formulations Of The Invention

In another embodiment, the topical carrier used in the topical formulations of the invention is an aqueous solution or suspension, preferably, an aqueous solution. Well-known ophthalmic solutions and suspensions are suitable topical carriers for use in the invention. Suitable aqueous topical formulations for use in the invention are disclosed in REMINGTON: THE SCIENCE AND PRACTICE OF PHARMACY 1563-1576 (Alfonso R. Gennaro ed. 19th ed. 1995). Other suitable aqueous topical carrier systems are disclosed in U.S. Patent Nos. 5,424,078 (issued Jun. 13, 1995); 5,736,165 (issued Apr. 7, 1998); 6,194,415 (issued Feb. 27, 2001); 6,248,741 (issued Jun. 19, 2001); 6,465,464 (issued Oct. 15, 2002).

The pH of the aqueous topical formulations of the invention are preferably within the range of from about 6 to about 8, more preferably, of from about 6.3 to about 6.5. To stabilize the pH, preferably, an effective amount of a buffer is included. In one embodiment, the buffering agent is present in the aqueous topical formulation in an amount of from about 0.05 to about 1 weight percent of the formulation. Acids or bases can be used to adjust the pH as needed. Suitable buffering agents are listed below in Section 1.3.3.

Tonicity-adjusting agents can be included in the aqueous topical formulations of the invention. Examples of suitable tonicity-adjusting agents include, but are not limited to, sodium chloride, potassium chloride, mannitol, dextrose, glycerin, and propylene glycol. The amount of the tonicity agent can vary widely depending on the formulation's desired properties. In one embodiment, the tonicity-adjusting agent is present in the aqueous topical formulation in an amount of from about 0.5 to about 0.9 weight percent of the formulation.

Preferably, the aqueous topical formulations of the invention have a viscosity in the range of from 0.015 to 0.025 Pa.s (about 15 cps to about 25 cps). The viscosity of aqueous solutions of the invention can be adjusted by adding viscosity adjusting agents, for examples, but not limited to, polyvinyl alcohol, povidone, hydroxypropyl methyl cellulose, poloxamers, carboxymethyl cellulose, or hydroxyethyl cellulose.

In a preferred embodiment, the aqueous topical formulation of the invention is isotonic saline comprising a preservative, such as benzalkonium chloride or chlorine dioxide, a viscosity-adjusting agent, such as polyvinyl alcohol, and a buffer system such as sodium citrate and citric acid.

### 1.3.3 Excipients

The topical formulations of the invention can comprise pharmaceutically acceptable excipients such as those listed in REMINGTON: THE SCIENCE AND PRACTICE OF PHARMACY 866-885(Alfonso R. Gennaro ed. 19th ed. 1995; Ghosh, T. K.; et al. TRANSDERMAL AND TOPICAL DRUG DELIVERY SYSTEMS (1997), including, but not limited to, protectives, adsorbents, demulcents, emollients, preservatives, antioxidants, moisturizers, buffering agents, solubilizing agents, skin-penetration agents, and surfactants.

Suitable protectives and adsorbents include, but are not limited to, dusting powders, zinc sterate, collodion, dimethicone, silicones, zinc carbonate, aloe vera gel and other aloe products, vitamin E oil, allatoin, glycerin, petrolatum, and zinc oxide.

Suitable demulcents include, but are not limited to, benzoin hydroxypropyl cellulose, hydroxypropyl methylcellulose, and polyvinyl alcohol.

Suitable emollients include, but are not limited to, animal and vegetable fats and oils, myristyl alcohol, alum, and aluminum acetate.

Suitable preservatives include, but are not limited to, quaternary ammonium compounds, such as benzalkonium chloride, benzethonium chloride, cetrimide, dequalinium chloride, and cetylpyridinium chloride; mercurial agents, such as phenylmercuric nitrate, phenylmercuric acetate, and thimerosal; alcoholic agents, for example, chlorobutanol, phenylethyl alcohol, and benzyl alcohol; antibacterial esters, for example, esters of parahydroxybenzoic acid; and other anti-microbial agents such as chlorhexidine, chlorocresol, benzoic acid and polymyxin.

Chlorine dioxide (ClO₂), preferably, stabilized chlorine dioxide, is a preferred preservative for use with topical formulations of the invention. The term "stabilized chlorine dioxide" is well known in the industry and by those skilled in the art. Stabilized chlorine dioxide includes one or more chlorine dioxide precursors such as one or more chlorine dioxide-containing complexes and/or one or more chlorite-containing components and/or one or more other entities capable of decomposing or being decomposed in an aqueous medium to form chlorine dioxide. U.S. Patent No. 5,424,078 (issued Jun. 13,1995), discloses a form of stabilized chlorine dioxide and a method for producing same, which can be used as a preservative for aqueous ophthalmic solutions and is useful in topical formulations of the invention. The manufacture or production of certain stabilized chlorine dioxide products is described in U.S. Pat. No. 3,278,447. A commercially available stabilized chlorine dioxide which can be utilized in the practice of the present invention is the proprietary stabilized chlorine dioxide of BioCide International, Inc. of Norman, OK, sold under the trademark Purogene™ or Purite™. Other suitable stabilized chlorine dioxide products include that sold under the trademark DuraKlor by Rio Linda Chemical Company, Inc., and that sold under the trademark Antheium Dioxide by International Dioxide, Inc.

Suitable antioxidants include, but are not limited to, ascorbic acid and its esters, sodium bisulfite, butylated hydroxytoluene, butylated hydroxyanisole, tocopherols, and chelating agents like EDTA and citric acid.

Suitable moisturizers include, but are not limited to, glycerin, sorbitol, polyethylene glycols, urea, and propylene glycol.

Suitable buffering agents for use in the invention include, but are not limited to, acetate buffers, citrate buffers, phosphate buffers, lactic acid buffers, and borate buffers.

Suitable solubilizing agents include, but are not limited to, quaternary ammonium chlorides, cyclodextrins, benzyl benzoate, lecithin, and polysorbates.

Suitable skin-penetration agents include, but are not limited to, ethyl alcohol, isopropyl alcohol, octylphenylpolyethylene glycol, oleic acid, polyethylene glycol 400, propylene glycol, N-decylmethylsulfoxide, fatty acid esters (e.g., isopropyl myristate, methyl laurate, glycerol monooleate, and propylene glycol monooleate); and N-methyl pyrrolidone.

### 1.3.4 Pharmaceutical Additives

The topical formulations of the invention can include pharmaceuticals or their pharmaceutically acceptable salts, for example, but not limited to, topical corticosteroids and other anti-inflammatory agents, such as betamethasone, diflorasone, amcinonide, fluocinolone, mometasone, hydrocortisone, prednisone, and triamcinolone; local anesthetics and analgesics, such as camphor, menthol, lidocaine, and dibucaine, and pramoxine; antifungals, such as ciclopirox, chloroxylenol, triacetin, sulconazole, nystatin, undecylenic acid, tolnaftate, miconizole, clotrimazole, oxiconazole, griseofulvin, econazole, ketoconozole, and amphotericin B; antibiotics and anti-infectives, such as mupirocin, erythromycin, clindamycin, gentamicin, polymyxin, bacitracin, and silver sulfadiazine; and antiseptics, such as iodine, povidine-iodine, benzalkonium chloride, benzoic acid, chlorhexidine, nitrofurazine, benzoyl peroxide, hydrogen peroxide, hexachlorophene, phenol, resorcinol, and cetylpyridinium chloride.

### 1.4 DOSAGE

Dosages and dosing frequency will be determined by a trained medical professional depending on the activity of the compound underlying the composition of the invention, the characteristics of the particular topical formulation, and the identity and severity of the dermatologic disorder treated or prevented.

In general, a compound described above is present in a formulation of the invention in an amount of from about 0.01 percent to about 5 percent of the total weight of the formulation, preferably, of from about 0.05 percent to about 1 percent, more preferably, of from about 0.1 percent to about 0.2 percent of the total weight of the formulation.

To treat or prevent the inflammatory skin disorders specified in the appended claims, the topical formulations of the invention are topically applied directly to the affected area in any conventional manner well known in the art. For example, by dropper or applicator stick, as a mist via an aerosol applicator, via an intradermal or transdermal patch, or by simply spreading a formulation of the invention onto the affected area with fingers. Generally the amount of a topical formulation of the invention applied to the affected skin area ranges from about 0.1 g/cm² of skin surface area to about 5 g/cm², preferably, 0.2 g/cm² to about 0.5 g/cm² of skin surface area. Typically, one to four applications per day are recommended during the term of treatment.

### 1.5 USE OF TOPICAL FORMULATIONS OF THE INVENTION IN COMBINATION WITH OTHER SKIN-DISORDER TREATMENTS

The formulations of the invention can be used in combination with other treatments and medications to provide more effective treatment or prevention of the inflammatory skin disorders and symptoms associated therewith. The topical formulations of the invention can be used in combination with treatment regimens and medications well known for treatment of dermatologic disorders, such as those disclosed in THE MERCK MANUAL 811-830 (Keryn A.G. Lane et al. eds. 17th ed. 2001).

Using a formulation of the invention in combination with another medicament or treatment means administering a formulation of the invention and the other medicament or treatment to a subject in a sequence and within a time interval such that they can act together to treat or prevent the inflammatory skin disorders and symptoms associated therewith. For example, the compounds underlying the compositions of the invention can be administered at the same time as the other medicament in the same or separate formulations or at different times.

Any suitable route of administration can be employed to deliver the additional treatment or medication including, but not limited to, oral, intraoral, rectal, parenteral, topical, epicutaneous, transdermal, subcutaneous, intramuscular, intranasal, sublingual, buccal, intradural, intraocular, intrarespiratory, or nasal inhalation. Thus, the formulations of the invention can be administered together or at separate times with other medications or treatments.

In one embodiment, the topical formulations of the invention are used in combination with systemic administration of antibiotics or retinoids including, but not limited to, orally dosed antibiotics, such as tetracycline, minocin, minocycline, erythromycin, and doxycycline, and orally dosed retinoids such as isotretinoins (e.g., Accutane or Roaccutance).

In another embodiment, the topical formulations of the invention are used in combination with other topical treatments including, but not limited to, topical formulations consisting of metronidizole, hydrogen peroxide, benzoyl peroxide, lipoic acid, and azelaic acid, and sulfur preparations; topically dosed antibiotics, such as metronidazole, clindamycin, and erythromycin; topical retinoids such as tretinoin, adapalene, tazarotene; or topical steroids.

In another embodiment, the topical formulations of the invention are used in combination with mixed light pulse therapy (photoderm), pulsed dye laser treatment, or electrosurgery.

### 1.6 ARTICLE OF MANUFACTURE

Another aspect of the invention is an article of manufacture that comprises a topical formulation of the invention in a suitable container with labeling and instructions for use. The container can be a dropper or tube with a suitable small orifice size, such as an extended tip tube made of any pharmaceutically suitable material.

The topical formulations of the invention can be filled and packaged into a plastic squeeze bottle or tube. Suitable container-closure systems for packaging topical formulations of the invention are commercially available for example, from Wheaton Plastic Products, 1101 Wheaton Avenue, Millville, NJ 08332.

Preferably, instructions are packaged with the formulations of the invention, for example, a pamphlet or package label. The labeling instructions explain how to administer topical formulations of the invention, in an amount and for a period of time sufficient to treat or prevent the inflammatory skin disorders and symptoms associated therewith. The labeling instructions are an important aspect of the invention in that before a composition can be approved for any particular use, it must be approved for marketing by the United States Food and Drug Administration. Part of that process includes providing a label that will accompany the pharmaceutical composition that is ultimately sold. Preferably, the label includes the dosage and administration instructions, the topical formulation's composition, the clinical pharmacology, drug resistance, pharmacokinetics, absorption, bioavailability, and contraindications.

### 1.7 EXAMPLES

The following examples are provided for illustrative purposes only and are not to be construed as limiting the invention's scope in any manner.

### 1.7.1 Example 1: Synthesis of (5-Bromo-quinoxalin-6-yl)-(4,5-dihydro-1H-imidazol-2-yl)-amine

To a stirred solution of 6-amino-5-bromoquinoxaline hydrobromide (10 g) in distilled water (150 ml) is added thiophosgene (3 ml). The solution is stirred for two hours at room temperature and the resultant precipitate is collected by filtration, washed with water, and dried to afford 5-bromo-6-isothiocyanato-quinoxaline.

The 5-bromo-6-isothiocyanato-quinoxaline (3.5 g.) is directly dissolved in benzene (400 ml) and added dropwise to a well-stirred solution of ethylene diamine (15 g.) in benzene (50 ml). During a period of about two hours, an oil separates as a lower layer. The upper benzene layer is poured off and the oil is washed with diethyl ether and then dissolved in methanol (500 ml). The methanolic solution is refluxed until hydrogen sulfide evolution ceases. The methanolic solution is concentrated *in vacuo* to a volume of approximately 100 ml upon which a yellow solid precipitates. The precipitate is collected by filtration and recrystallized from methanol to afford of (5-Bromo-quinoxalin-6-yl)-(4,5-dihydro-1H-imidazol-2-yl)-amine: m.p. 250-251 C.

### 1.7.2 Example 2

An aqueous solution topical formulation of the invention comprises (5-Bromo-quinoxalin-6-yl)-(4,5-dihydro-1H-imidazol-2-yl)-amine-L-tartrate (brimonidine tartrate) (0.15 wt. %); Purite® (0.005%) (stabilized chlorine dioxide) as a preservative; and the inactive ingredients: boric acid; calcium chloride; magnesium chloride; potassium chloride; purified water; sodium borate; sodium carboxymethylcellulose; sodium chloride; with hydrochloric acid and/or sodium hydroxide to adjust the pH to 5.6 to 6.6. The osmolality is in the range of 250-350 mOsmol/kg.

### 1.7.3 Example 3

A aqueous solution topical formulation of the invention comprises (5-Bromo-quinoxalin-6-yl)-(4,5-dihydro-1H-imidazol-2-yl)-amine-L-tartrate, (brimonidine tartrate) (0.15 wt. %); benzalkonium chloride (0.005 wt. %) as a preservative; and the inactive ingredients: boric acid; calcium chloride; magnesium chloride; potassium chloride; purified water; sodium borate; sodium carboxymethylcellulose; sodium chloride; with hydrochloric acid and/or sodium hydroxide to adjust the pH to 5.6 to 6.6. The osmolality is in the range of 250-350 mOsmol/kg.

### 1.7.4 Example 4

A possible cream topical formulation of the invention is described in the Table below.

**Possible Cream Formulation Of The Invention (Hydrophilic Ointment USP)**

| Ingredient | Weight Percent |
|---|---|
| Brimonidine tartrate | 0.15% |
| Stearic acid | 7% |
| Stearyl alcohol | 5% |
| Cetyl alcohol | 2% |
| Glycerin | 10% |
| Sodium lauryl sulfate | 1% |
| Propylparaben | 0.05% |
| Methylparaben | 0.25% |
| Disodium edetate | 0.055 |
| Distilled water | QS |

Melt the stearyl alcohol and the white petrolatum on a steam bath, and warm to about 75 degrees C. Add the other ingredients, previously dissolved in the water and warmed to 75 degrees C, and stir the mixture until it congeals. With stirring, allow the mixture to cool and add brimonidine tartrate as a concentrated solution.

### 1.7.5 Example 5

A possible ointment topical formulation of the invention is described in the Table below.

**Possible Ointment Formulation of the Invention (Hydrophilic Ointment USP)**

| Ingredients | Weight |
|---|---|
| Brimonidine tartrate | 10g |
| Cholesterol | 30g |
| Stearyl Alcohol | 30g |
| White Wax | 80g |
| White Petrolatum | 850g |

Mix the stearyl alcohol and white wax together on a steam bath, then add the cholesterol and stir until it completely dissolves. Add the white petrolatum and mix. Remove from the bath, and stir until the mixture congeals. Continue stirring and add brimonidine tartrate as a concentrated slurry.

### 1.7.6 Example 6

A possible gel formulation of the invention is described in the table below.

**Possible Gel Formulation of the Invention**

| Ingredients | Weight % |
|---|---|
| Brimonidine tartrate | 1.0% |
| Methylparaben NF | 0.15% |
| Propylparaben NF | 0.03% |
| Hydroxyethylcellulose NF | 1.25% |
| Disodium Edetate USP | 0.05% |
| Purified Water, USP | QS 100% |

### 1.7.7 Example 7

A possible gel formulation of the invention is described in the Table below.

**Possible Gel Formulation of the Invention**

| Ingredients | Weight % |
|---|---|
| Brimonidine tartrate | 1.0% |
| Methylparaben | 0.20% |
| Propylparaben | 0.05% |
| Carbomer 934P NF | 1.0% |
| Sodium Hydroxide | QS pH 7 |
| Purified Water USP | QS 100% |

The ingredients are mixed together and aqueous sodium hydroxide is slowly added to the mixture until a pH of about 7 is reached and the gel is formed.

### 1.7.8 Example 8

A possible gel formulation of the invention is described in the Table below.

**Possible Gel Formulation of the invention**

| Ingredients | Weight % |
|---|---|
| Brimonidine tartrate | 1.0% |
| Methylparaben | 0.2% |
| Propylparaben | 0.05% |
| "CARBOPOL®" | 1.0% |
| Triethanolamine | QS pH 7 |
| Water | QS 100 % |

The ingredients are mixed together and stirred. Triethanolamine is added until a pH of about 7 is attained.

### 1.8 DEFINITIONS

As used herein, the terms "an inflammatory skin disorder" and "an inflammatory dermatologic disorder" mean dermatitis, such as contact dermatitis, atopic dermatitis, seborrheic dermatitis, nummular dermatitis, generalized exfoliative dermatitis, statis dermatitis, lichen simplex chronicus; disorders of hair follicles and sebaceous glands, such as acne, rhinophyma, perioral dermatitis, and pseudofolliculitis barbae; and inflammatory reactions, such as drug eruptions, erythema multiforme, erythema nodosum, and granuloma annulare. In a preferred embodiment, the topical formulations of the invention are used to treat or prevent inflammatory dermatologic disorders of the face.

The phrase "pharmaceutically acceptable salt(s)", as used herein, means those salts of compounds underlying the compositions of the invention that are safe and effective for topical use in mammals and that possess the desired biological activity. Pharmaceutically acceptable salts include salts of acidic or basic groups present in compounds of the invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts. Certain compounds underlying the compositions of the invention can form pharmaceutically acceptable salts with various amino acids. Suitable base salts include, but are not limited to, aluminum, calcium, lithium, magnesium, potassium, sodium, zinc, and diethanolamine salts. For a review on pharmaceutically acceptable salts see BERGE ET AL., 66 J. PHARM. SCI. 1-19 (1977).

The term "pharmaceutically acceptable topical formulation" as used herein means any formulation which is pharmaceutically acceptable for topical delivery of the compounds underlying the compositions of the invention. According to the invention, a "topical formulation" will comprise at least a compound underlying the compositions of the invention. The choice of topical formulation will depend on several factors, including the nature of the symptoms to be treated or prevented, the physiochemical characteristics of the particular compound of the invention and of other excipients present, their stability in the formulation, available manufacturing equipment, and cost constraints.

As used herein, a "therapeutically effective amount of a compound underlying the compositions of the invention" means the minimum amount of the compound that is effective to treat or prevent an inflammatory dermatologic disorder.

As used herein, the term "subject" means any animal, preferably a mammal, to which will be or has been administered topical formulations of the invention. The term "mammal" as used herein, encompasses any mammal. Examples of mammals include, but are not limited to, cows, horses, sheep, pigs, cats, dogs, mice, rats, rabbits, guinea pigs, monkeys, humans etc., more preferably, a human. Preferably, a subject is in need of treatment or prevention of an inflammatory skin disorder and symptoms associated therewith.

The term "analog" refers to a chemical compound that is structurally similar to a parent compound and has chemical properties or pharmaceutical activity in common with the parent compound. Analogs include, but are not limited to, homologs, i.e., where the analog differs from the parent compound by one or more carbon atoms in series; positional isomers; compounds that differ by interchange of one or more atoms by a different atom, for example, replacement of a carbon atom with an oxygen, sulfur, or nitrogen atom; and compounds that differ in the identity of one or more functional groups, for example, the parent compound differs from its analog by the presence or absence of one or more suitable substituents. Suitable substituents include, but are not limited to, (C₁-C₈)alkyl; (C₁-C₈)alkenyl; (C₁-C₈)alkynyl: aryl; (C₂-C₅)heteroaryl; (C₁-C₆)heterocycloalkyl; (C₃-C₇)cycloalkyl; O-(C₁-C₈)alkyl; O-(C₁-C₈)alkenyl; O-(C₁-C₈)alkynyl; O-aryl; CN; OH; oxo; halo, C(O)OH; COhalo; O(CO)halo; CF₃, N₃; NO₂, NH₂; NH((C₁-C₈)alkyl); N((C₁-C₈)akl)₂; NH(aryl); N(aryl)₂N((C₁-C₈)alkyl)(aryl); (CO)NH₂; (CO)NH((C₁-C₈)alkyl); (CO)N((C₁-C₈)alkyl)₂; (CO)NH(aryl); (CO)N(aryl)₂; O(CO)NH₂; NHOH; NOH((C₁-C₈)alkyl); NOH(aryl); O(CO)NH(C₁-C₈)alkyl); O(CO)N((C₁-C₈)alkyl)₂; O(CO)NH(aryl); O(CO)N(aryl)₂; CHO; CO((C₁-C₈)alkyl); CO(aryl); C(O)O((C₁-C₈)alkyl); C(O)O(aryl); O(CO)((C₁-C₈)alkyl); O(CO)aryl); O(CO)O((C₁-C₈)alkyl); O(CO)O(aryl); S-(C₁-C₈)alkyl; S-(C₁-C₈)alkenyl; S-(C₁-C₈)alkynyl; S-aryl; S(O)-(C₁-C₈)alkyl; S(O)-(C₁-C₈)alkenyl; S(O)-(C₁-C₈)alkynyl; and S(O)-aryl; S(O)₂-C₁-C₈)alkyl; S(O)₂-(C₁-C₈)alkenyl; S(O)₂-(C₁-C₈)alkynyl; and S(O)₂-aryl. One of skill in the art can readily choose a suitable substituent based upon the stability and pharmacological activity of the compound underlying the composition of the invention.

The term "alkyl" means a saturated, monovalent, unbranched or branched hydrocarbon chain. Examples of alkyl groups include, but are not limited to, (C₁-C₃)alkyl groups, such as methyl, ethyl, propyl, isopropyl and (C₄-C₈)alkyl groups, such as 2-methyl-1-propyl, 2-methyl-2-propyl, 2-methyl-1-butyl, 3-methyl-1-butyl, 2-methyl-3-butyl, 2,2-dimethyl-1-propyl, 2-methyl-pentyl, 3-methyl-1-pentyl, 4-methyl-1-pentyl, 2-methyl-2-pentyl, 3- methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, and hexyl, heptyl, and octyl. An alkyl group can be unsubstituted or substituted with one or two suitable attachments.

The term "alkynyl" means a monovalent, unbranched or branched hydrocarbon chain having one or more double bonds therein. The double bond of an alkenyl group can be unconjugated or conjugated to another unsaturated group. Suitable alkenyl groups include, but are not limited to (C₂-C₈)alkenyl groups, such as vinyl, allyl, butenyl, pentenyl, hexenyl, butadienyl, pentadienyl, hexadienyl, 2-ethylhexenyl,2-propyl-2-butenyl,4-(2-methyl-3-butene)-pentenyl. An alkenyl group can be unsubstituted or substituted with one or two suitable substituents.

The term"alkynyl" means monovalent, unbranched or branched hydrocarbon chain having one or more triple bonds therein. The triple bond of an alkynyl group can be unconjugated or conjugated to another unsaturated group. Suitable alkynyl groups include, but are not limited to, (C₂-C₈)alkynyl groups, such as ethynyl, propynyl, butynyl, pentynyl, hexynyl, methylpropynyl, 4-methyl-1-butynyl,4-propyl-2-pentynyl, and 4-butyl-2-hexynyl. An alkynyl group can be unsubstituted or substituted with one or two suitable substituents.

The term "aryl" means a monocyclic or polycyclic-aromatic group comprising carbon and hydrogen atoms. Examples of suitable aryl groups include, but are not limited to, phenyl, tolyl, anthracenyl, fluorenyl, indenyl, azulenyl, and naphthyl, as well as benzo-fused carbocyclic moieties such as 5,6,7,8-tetrahydronaphthyl. An aryl group can be unsubstituted or substituted with one or two suitable substituents. Preferably, the aryl group is a monocyclic ring, wherein the ring comprises 6 carbon atoms, referred to herein as "(C₆)aryl".

The term "heteroaryl" means a monocyclic- or polycyclic aromatic ring comprising carbon atoms, hydrogen atoms, and one or more heteroatoms, preferably, 1 to 3 heteroatoms, independently selected from nitrogen, oxygen, and sulfur. As is well known to those skilled in the art, heteroaryl rings have less aromatic character than their all-carbon counter parts. Thus, for the purposes of the invention, a heteroaryl group need only have some degree of aromatic character. Illustrative examples of heteroaryl groups include, but are not limited to, pyridinyl, pyridazinyl, pyrimidyl, pyrazyl, triazinyl, pyrrolyl, pyrazolyl, imidazolyl, (1,2,3,)- and (1,2,4)-triazolyl, pyrazinyl, pyrimidinyl, tetrazolyl, furyl, thienyl, isoxazolyl, thiazolyl, phenyl, isoxazolyl, and oxazolyl. A heteroaryl group can be unsubstituted or substituted with one or two suitable substituents. Preferably, a heteroaryl group is a monocyclic ring, wherein the ring comprises 2 to 5 carbon atoms and 1 to 3 heteroatoms, referred to herein as "(C₂-C₅)heteroaryl".

The term "cycloalkyl" means a non-aromatic, monocyclic or polycyclic ring comprising carbon and hydrogen atoms. A cycloalkyl group can have one or more carbon-carbon double bonds in the ring so long as the ring is not rendered aromatic by their presence. Examples of cycloalkyl groups include, but are not limited to, (C₃-C₇)cycloalkyl groups, such as cyclopropyl, cyclobutyl, cyclopentyl, cyclohexyl, and cycloheptyl, and saturated cyclic and bicyclic terpenes and (C₃-C₇)cycloalkenyl groups, such as cyclopropenyl, cyclobutenyl, cyclopentenyl, cyclohexenyl, and cycloheptenyl, and unsaturated cyclic and bicyclic terpenes. A cycloalkyl group can be unsubstituted or substituted by one or two suitable substituents. Preferably, the cycloalkyl group is a monocyclic ring or bicyclic ring.

The term "heterocycloalkyl" means a non-aromatic monocyclic or polycyclic ring comprising carbon and hydrogen atoms and at least one heteroatom, preferably, 1 to 4 heteroatoms selected from nitrogen, oxygen, and sulfur. A heterocycloalkyl group can have one or more carbon-carbon double bonds or carbon-heteroatoms double bonds in the ring as long as the ring is not rendered aromatic by their presence. Examples of heterocycloalkyl groups include aziridinyl, pyrrolidinyl, pyrrolidino, piperidinyl, piperidino, piperazinyl, piperazino, morpholinyl, morpholino, thiomorpholinyl, thiomorpholino, tetrahydrofuranyl, tetrahydrothiofuranyl, tetrahydropyranyl, and pyranyl. A heterocycloalkyl group can be unsubstituted or substituted with one or two suitable substituents. Preferably, the heterocycloalkyl group is a monocyclic or bicyclic ring, more preferably, a monocyclic ring, wherein the ring comprises from 2 to 6 carbon atoms and from 1 to 3 heteroatoms, referred to herein as (C₁-C₆)heterocycloalkyl.

The term "halogen" means fluorine, chlorine, bromine, or iodine. Correspondingly, the term "halo" means fluoro, chloro, bromo, and iodo.

The term "derivative" refers to an analog, as defined above, that is synthesized in one or more chemical reactions from its parent compound.

As used herein, the term "hydrate" means a compound underlying the compositions of the invention, or a pharmaceutically acceptable salt thereof that further includes a stoichiometric or non-stoichiometric amount of water bound to it by non-covalent intermolecular forces.

In one embodiment, "treatment" or "treating" refers to an amelioration, prophylaxis, or reversal of a disease or disorder, or at least one discernible symptom thereof. For example, treating an inflammatory skin disorder by lessening the redness of the skin. In another embodiment, "treatment" or "treating" refers to an amelioration, prophylaxis, or reversal of at least one measurable physical parameter related to the disease or disorder being treated, not necessarily discernible in or by the mammal. In yet another embodiment, "treatment" or "treating" refers to inhibiting or slowing the progression of a disease or disorder, either physically, e.g., stabilization of a discernible symptom, physiologically, e.g., stabilization of a physical parameter, or both. In yet another embodiment, "treatment" or "treating" refers to delaying the onset of a disease or disorder.

In certain embodiments, the compounds underlying the compositions of the invention are administered as a preventative measure. As used herein, "prevention" or "preventing" refers to a reduction of the risk of acquiring a given disease or disorder. In a preferred mode of the embodiment, the compounds underlying the compositions of the invention are administered as a preventative measure to a subject having a predisposition to the inflammatory skin disorder even though symptoms of the disorder are absent or minimal.

As used herein, "carbomer" is the USP designation for various polymeric acids that are dispersible but insoluble in water. When the acid dispersion is neutralized with a base a clear, stable gel is formed. Carbomer 934P is physiologically inert and is not a primary irritant or sensitizer. Other carbomers include 910, 940, 941, and 1342.

One of ordinary skill in the art can make many variations and modifications to the above-described embodiments of the invention without departing from the scope of the appended claims.

## Claims

1. A composition comprising:
at least one of an α₂ adrenergic receptor agonist and a pharmaceutically acceptable salt thereof; and
a pharmaceutically acceptable carrier;
for use in the treatment or prevention of an inflammatory skin disorder and the symptoms associated therewith,
wherein said composition is to be topically administered to the skin of a patient in need of such treatment or prevention,
wherein the at least one of an α₂ adrenergic receptor agonist and a pharmaceutically acceptable salt thereof is selected from the group consisting of brimonidine and oxymetazoline, and
wherein said inflammatory skin disorder is selected from the group consisting of dermatitis, including contact dermatitis, atopic dermatitis, seborrheic dermatitis, nummular dermatitis, generalized exfoliative dermatitis, statis dermatitis, lichen simplex chronicus; disorders of hair follicles and sebaceous glands, including acne, rhinophyma, perioral dermatitis, and pseudofolliculitis barbae; and inflammatory reactions, including drug eruptions, erythema multiforme, erythema nodosum, and granuloma annulare.

2. Use of a composition comprising:
at least one of an α₂ adrenergic receptor agonist and a pharmaceutically acceptable salt thereof; and
a pharmaceutically acceptable carrier;
for the preparation of a medicament for treating or preventing an inflammatory skin disorder and the symptoms associated therewith,
wherein said composition is to be topically administered to the skin of a patient in need of such treatment or prevention,
wherein the at least one of an α₂ adrenergic receptor agonist and a pharmaceutically acceptable salt thereof is selected from the group consisting of brimonidine and oxymetazoline, and
wherein said inflammatory skin disorder is selected from the group consisting of dermatitis, including contact dermatitis, atopic dermatitis, seborrheic dermatitis, nummular dermatitis, generalized exfoliative dermatitis, statis dermatitis, lichen simplex chronicus; disorders of hair follicles and sebaceous glands, including acne, rhinophyma, perioral dermatitis, and pseudofolliculitis barbae; and inflammatory reactions, including drug eruptions, erythema multiforme, erythema nodosum, and granuloma annulare.

3. A use or the composition for use according to claim 1 or claim 2, wherein the at least one of an α₂ adrenergic receptor agonist and a pharmaceutically acceptable salt thereof is to be administered in an amount sufficient to decrease blood flow through the small arteries or arterioles of the skin of the patient.

4. A use or the composition for use according to claim 1 or claim 2, wherein the pharmaceutically acceptable carrier is selected from the group consisting of sprays, mists, aerosols, solutions, lotions, gels, creams, ointments, pastes, unguents, emulsions, and suspensions.

5. A use or the composition for use according to claim 1 or claim 2, wherein the composition acts locally in the skin of the patient.

6. The composition for use according to claim 4, wherein the pharmaceutically acceptable carrier is an aqueous gel comprising water, and a water-gelling amount of a pharmaceutically acceptable gelling agent selected from the group consisting of carbomers, glycerine polyacrylate, and mixtures thereof, the topical composition having a physiologically acceptable pH.

7. The composition for use according to claim 4, wherein the pharmaceutically acceptable carrier is at least one of a cream and an ointment comprising stearic acid, stearyl alcohol, cetyl alcohol, glycerin, and water, the topical composition having a physiologically acceptable pH.

8. The composition for use according to any one of claims 4, 6 or 7, wherein the at least one of an α₂ adrenergic receptor agonist and a pharmaceutically acceptable salt thereof is present in an amount in the range of about 0.01 to 5 weight percent.

9. The composition for use according to any one of claims 4, 6 or 7, wherein the pH value of the composition is in the range of about 5 to 8.

10. The composition for use according to claim 6 or 7, further comprising a preservative.

11. The composition for use according to claim 6 or 7, further comprising a local anesthetic.

12. The composition for use according to claim 6 or 7, further comprising a skin humectant.

13. A package including a composition as defined in claim 1, said package comprising a container and instructions for use of the composition for topically treating or preventing an inflammatory skin disorder as defined in claim 1.

## Patentansprüche

1. Zusammensetzung umfassend:
mindestens einen ausgewählt aus einem α₂ adrenergen Rezeptoragonisten und einem pharmazeutisch akzeptablen Salz davon; und
einen pharmazeutisch akzeptablen Träger;
zur Verwendung in der Behandlung oder Vorbeugung einer entzündlichen Hauterkrankung und dazugehörigen Symptomen,
wobei die Zusammensetzung topisch auf die Haut eines Patienten, der einer solchen Behandlung oder Vorbeugung bedarf, aufgetragen wird,
wobei der mindestens eine ausgewählt aus einem α₂ adrenergen Rezeptoragonisten und einem pharmazeutisch akzeptablen Salz davon ausgewählt ist aus der Gruppe bestehend aus Brimonidin und Oxymetazolin, und
wobei die entzündliche Hauterkrankung ausgewählt ist aus der Gruppe bestehend aus Dermatitis einschließlich Kontaktdermatitis, atopische Dermatitis, seborrhöische Dermatitis, nummulare Dermatitis, generalisierte exfoliative Dermatitis, varikoses Ekzem, Lichen simplex chronicus; Erkrankungen der Haarfollikel und Talgdrüsen einschließlich Akne, Rhinophyma, perioraler Dermatitis und Pseudofolliculitis barbae; und entzündliche Reaktionen einschließlich Arzneimittelexanthemen, Erythema multiforme, Erythema nodosum, und Granuloma annulare.

2. Verwendung einer Zusammensetzung umfassend:
mindestens einen ausgewählt aus einem α₂ adrenergen Rezeptoragonisten und einem pharmazeutisch akzeptablen Salz davon; und
einen pharmazeutisch akzeptablen Träger;
zur Herstellung eines Medikaments zur Behandlung oder Vorbeugung einer entzündlichen Hauterkrankung und dazugehörigen Symptomen,
wobei die Zusammensetzung topisch auf die Haut eines Patienten, der einer solchen Behandlung oder Vorbeugung bedarf, aufgetragen wird,
wobei der mindestens eine ausgewählt aus einem α₂ adrenergen Rezeptoragonisten und einem pharmazeutisch akzeptablen Salz davon ausgewählt ist aus der Gruppe bestehend aus Brimonidin und Oxymetazolin, und
wobei die entzündliche Hauterkrankung ausgewählt ist aus der Gruppe bestehend aus Dermatitis einschließlich Kontaktdermatitis, atopische Dermatitis, seborrhöische Dermatitis, nummulare Dermatitis, generalisierte exfoliative Dermatitis, varikoses Ekzem, Lichen simplex chronicus; Erkrankungen der Haarfollikel und Talgdrüsen einschließlich Akne, Rhinophyma, perioraler Dermatitis und Pseudofolliculitis barbae; und entzündliche Reaktionen einschließlich Arzneimittelexanthemen, Erythema multiforme, Erythema nodosum, und Granuloma annulare.

3. Verwendung oder die Zusammensetzung zur Verwendung nach Anspruch 1 oder 2, wobei der mindestens eine ausgewählt aus einem α₂ adrenergen Rezeptoragonisten und einem pharmazeutisch akzeptablen Salz davon in einer Menge zu verabreichen ist, die ausreicht, Blutfluss durch die kleinen Arterien oder Arteriolen der Haut des Patienten zu verringern.

4. Verwendung oder die Zusammensetzung zur Verwendung nach Anspruch 1 oder 2, wobei der pharmazeutisch akzeptable Träger ausgewählt ist aus der Gruppe bestehend aus Sprays, Nebeln, Aerosolen, Lösungen, Lotionen, Gelen, Cremes, Balsamen, Pasten, Salben, Emulsionen, und Suspensionen.

5. Verwendung oder die Zusammensetzung zur Verwendung nach Anspruch 1 oder 2, wobei die Zusammensetzung lokal auf der Haut des Patienten wirkt.

6. Zusammensetzung zur Verwendung nach Anspruch 4, wobei der pharmazeutisch akzeptable Träger ein Wasser umfassendes wässriges Gel ist, und eine Wasser gelierende Menge eines pharmazeutisch akzeptablen Gelbildners ausgewählt aus der Gruppe bestehend aus Carbomeren, Glycerinpolyacrylat und Mischungen davon, wobei die topische Zusammensetzung einen physiologisch akzeptablen pH aufweist.

7. Zusammensetzung zur Verwendung nach Anspruch 4, wobei der pharmazeutisch akzeptable Träger mindestens einer aus einer Creme oder einer Salbe ist, die Stearinsäure, Stearylalkohol, Cetylalkohol, Glycerin und Wasser umfasst, wobei die topische Zusammensetzung einen physiologisch akzeptablen pH aufweist.

8. Zusammensetzung zur Verwendung nach einem der Ansprüche 4, 6 oder 7, wobei der mindestens eine ausgewählt aus einem α₂ adrenergen Rezeptoragonisten und einem pharmazeutisch akzeptablen Salz davon in einer Menge im Bereich von etwa 0.01 bis 5 Gewichtsprozent vorhanden ist.

9. Zusammensetzung zur Verwendung nach einem der Ansprüche 4, 6 oder 7, wobei der pH Wert der Zusammensetzung im Bereich von etwa 5 bis 8 liegt.

10. Zusammensetzung zur Verwendung nach Anspruch 6 oder 7, ferner umfassend ein Konservierungsmittel.

11. Zusammensetzung zur Verwendung nach Anspruch 6 oder 7, ferner umfassend ein Lokalanästhetikum.

12. Zusammensetzung zur Verwendung nach Anspruch 6 oder 7, ferner umfassend ein Hautbefeuchtungsmittel.

13. Packung, einschließlich einer Zusammensetzung wie in Anspruch 1 definiert, wobei die Packung einen Behälter und eine Gebrauchsanweisung für die Zusammensetzung zur topischen Behandlung oder Vorbeugung einer entzündlichen Hauterkrankung, wie in Anspruch 1 definiert, umfasst.

## Revendications

1. Composition comprenant :
au moins l'un d'un agoniste de récepteur α₂ adrénergique et d'un sel pharmaceutiquement acceptable de celui-ci ; et
un vecteur pharmaceutiquement acceptable ;
destinée à être utilisée dans le traitement ou la prévention d'un trouble cutané inflammatoire et des symptômes associés avec celui-ci,
où ladite composition est destinée à être administrée par voie topique à la peau d'un patient nécessitant un tel traitement ou une telle prévention,
où le au moins l'un d'un agoniste de récepteur α₂ adrénergique et d'un sel pharmaceutiquement acceptable de celui-ci est choisi dans le groupe consistant en la brimonidine et l'oxymétazoline, et
où ledit trouble cutané inflammatoire est choisi dans le groupe consistant en la dermatite, incluant la dermatite de contact, la dermatite atopique, la dermatite séborrhéique, la dermatite nummulaire, la dermatite exfoliative généralisée, la dermatite de stase, la névrodermite circonscrite ; les troubles des follicules pileux et des glandes sébacées, incluant l'acné, le rhinophyma, la dermatite périorale et le pseudosycosis de la barbe ; et les réactions inflammatoires, incluant les dermatites médicamenteuses, l'érythème polymorphe, l'érythème noueux et le granulome annulaire.

2. Utilisation d'une composition comprenant :
au moins l'un d'un agoniste de récepteur α₂ adrénergique et d'un sel pharmaceutiquement acceptable de celui-ci ; et
un vecteur pharmaceutiquement acceptable ;
pour la préparation d'un médicament pour traiter ou prévenir un trouble cutané inflammatoire et les symptômes associés avec celui-ci,
où ladite composition est destinée à être administrée par voie topique à la peau d'un patient nécessitant un tel traitement ou une telle prévention,
où le au moins l'un d'un agoniste de récepteur α₂ adrénergique et d'un sel pharmaceutiquement acceptable de celui-ci est choisi dans le groupe consistant en la brimonidine et l'oxymétazoline, et
où ledit trouble cutané inflammatoire est choisi dans le groupe consistant en la dermatite, incluant la dermatite de contact, la dermatite atopique, la dermatite séborrhéique, la dermatite nummulaire, la dermatite exfoliative généralisée, la dermatite de stase, la névrodermite circonscrite ; les troubles des follicules pileux et des glandes sébacées, incluant l'acné, le rhinophyma, la dermatite périorale et le pseudosycosis de la barbe ; et les réactions inflammatoires, incluant les dermatites médicamenteuses, l'érythème polymorphe, l'érythème noueux et le granulome annulaire.

3. Utilisation ou composition destinée à être utilisée selon la revendication 1 ou la revendication 2, où le au moins l'un d'un agoniste de récepteur α₂ adrénergique et d'un sel pharmaceutiquement acceptable de celui-ci est destiné à être administré en une quantité suffisante pour faire décroître le débit sanguin dans les petites artères ou les artérioles de la peau du patient.

4. Utilisation ou composition destinée à être utilisée selon la revendication 1 ou la revendication 2, où le vecteur pharmaceutiquement acceptable est choisi dans le groupe consistant en les sprays, les brouillards, les aérosols, les solutions, les lotions, les gels, les crèmes, les pommades, les pâtes, les onguents, les émulsions et les suspensions.

5. Utilisation ou composition destinée à être utilisée selon la revendication 1 ou la revendication 2, où la composition agit localement dans la peau du patient.

6. Composition destinée à être utilisée selon la revendication 4, où le vecteur pharmaceutiquement acceptable est un gel aqueux comprenant de l'eau, et une quantité gélifiant l'eau d'un agent gélifiant pharmaceutiquement acceptable choisi dans le groupe consistant en les carbomères, le polyacrylate de glycérine et leurs mélanges, la composition topique ayant un pH physiologiquement acceptable.

7. Composition destinée à être utilisée selon la revendication 4, où le vecteur pharmaceutiquement acceptable est au moins l'une d'une crème et d'une pommade comprenant de l'acide stéarique, de l'alcool stéarylique, de l'alcool cétylique, de la glycérine et de l'eau, la composition topique ayant un pH physiologiquement acceptable.

8. Composition destinée à être utilisée selon l'une quelconque des revendications 4, 6 ou 7, où le au moins l'un d'un agoniste de récepteur α₂ adrénergique et d'un sel pharmaceutiquement acceptable de celui-ci est présent en une quantité dans la plage d'environ 0,01 à 5 pourcent en poids.

9. Composition destinée à être utilisée selon l'une quelconque des revendications 4, 6 ou 7, où le pH de la composition est dans la plage d'environ 5 à 8.

10. Composition destinée à être utilisée selon la revendication 6 ou 7, comprenant en outre un conservateur.

11. Composition destinée à être utilisée selon la revendication 6 ou 7, comprenant en outre un anesthésique local.

12. Composition destinée à être utilisée selon la revendication 6 ou 7, comprenant en outre un humectant de la peau.

13. Ensemble incluant une composition comme défini dans la revendication 1, ledit ensemble comprenant un récipient et des instructions pour l'utilisation de la composition pour traiter ou prévenir par voie topique un trouble cutané inflammatoire comme défini dans la revendication 1.
